# EUROPEAN PATENT APPLICATION

(11) **EP 2 937 124 A1**
(43) Date of publication of application: **28.10.2015**
(21) Application number: 13865487.6
(22) Date of filing: 17.12.2013
(51) Int. Cl.: B01D 7/00, B01D 9/00, H01L 51/56, C07B 63/00

(54) **METHOD AND APPARATUS FOR PURIFYING ORGANIC MATERIAL USING IONIC LIQUID**

(30) Priority: 18.12.2012 KR 20120148718; 18.12.2012 KR 20120148719
(71) Applicant: Korea Institute of Industrial Technology, Cheonan-si, Chungcheongnam-do 331-822 (KR)
(72) Inventor: TAE WON, Kim, Gwangju 506-305 (KR); JONG HO, Lee, Gwangju 501-080 (KR); JAE CHEOL, Park, Jangseong-gun Jeollanam-do 515-804 (KR)
(74) Representative: Cabinet Chaillot
(86) International application number: PCT/KR2013/011757
(87) International publication number: WO 2014/098458

(57) **Abstract**

Disclosed is a simplified apparatus for purifying an organic material using an ionic liquid, including a sublimation unit for sublimating a raw organic material containing impurities for OLEDs by heating; a recrystallization unit for recrystallizing the sublimated gas of the organic material in the ionic liquid; and a control unit for controlling the operation of the sublimation unit and the recrystallization unit. Even when a very small amount of organic material is sublimated, the recrystallization thereof in the ionic liquid can be verified, thus easily checking the purification potential of the organic material using only a very small amount of organic material.

## Description

### Technical Field

The present invention relates to a method and apparatus for purifying an organic material using an ionic liquid process and, more particularly, to a simplified method and apparatus for purifying an organic material, suitable for use in screening the optimal purification combination between various ionic liquids and organic materials to be purified, wherein even when a very small amount of organic material for an organic light emitting diode (OLED) is used, whether any kind of ionic liquid is the most appropriate for recrystallizing the corresponding organic material can be quickly experimentally verified. Also, the present invention relates to a method and apparatus for purifying an organic material for OLEDs, in which a large amount of organic material may be purified using an ionic liquid as a filter.

### Background Art

Recently, OLEDs are receiving attention as a next-generation display device. This is because OLEDs obviate the high driving voltage that is required for inorganic LEDs, have the advantages of self-emission, a thin film form, a fast response rate, and a wide viewing angle, and make it easy to exhibit pleochroism due to a variety of organic compounds. Such OLEDs are devices in which holes and electrons are recombined in the emission layer to produce excitons in an excited state, and then light having a specific wavelength is emitted by energy from the produced excitons.

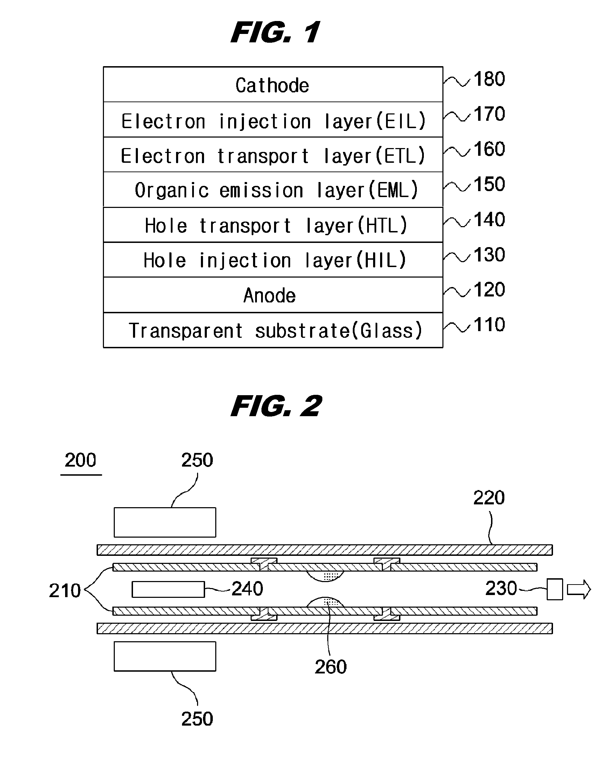

FIG. 1 illustrates the structure of an OLED. As illustrated in FIG. 1, the OLED has a stack structure configured such that an anode 120, a hole injection layer (HIL) 130, a hole transport layer (HTL) 140, an organic emission layer (EML) 150, an electron transport layer (ETL) 160, an electron injection layer (EIL) 170, and a cathode 180 are sequentially formed on a transparent substrate 110 such as glass.

The fabrication of the OLED having such a stack structure needs an organic material for light emission and charge transport. However, since such an organic material directly participates in the recombination of injected holes and electrons, as well as the injection of electrons and holes, the purity of the organic material is regarded as very important in terms of determining the color, emission efficiency, and lifespan of the OLED. Specifically, a small amount of impurities in the organic material may increase the probability of extinction of injected charges, thus decreasing the likelihood of recombination of holes and electrons, thereby lowering the emission efficiency. Furthermore, new energy levels are attained, due to the addition of impurities, thus deteriorating the purity of luminous color.

Thus, to achieve electroluminescent devices having high luminance, high efficiency and a long lifespan, the optimization of the structure of an electroluminescent device, the development of novel materials having superior hole (or electron) injection and transport properties and novel materials for organic emission layers, and improvements in the purity of organic materials for OLEDs are required.

Meanwhile, to purify organic materials for OLEDs, a recrystallization method, using either a solvent or sublimation, is typically employed. The recrystallization method using sublimation enables an organic material to be sublimated and recrystallized in a vacuum, and is thereby advantageous because there are no impurities. Hence, the sublimation method is generally adopted to purify an organic material for an organic electroluminescent device.

As used herein, the term "sublimation" refers to a gas-solid transition phenomenon at a temperature and pressure equal to or less than the triple point in a phase diagram. Any material that is pyrolyzed upon heating at atmospheric pressure does not decompose even at relatively high temperatures at low pressure below the triple point. In a sublimation system, in which it is possible to control the temperature gradient using such properties, a process of heating the mixed materials so as to be separated from impurities having different sublimation points while not being decomposed is referred to as a vacuum sublimation method. Such a vacuum sublimation method, which is a purely physical method, is advantageous because it does not depend on the use of assistant reagents or other chemical methods, thus avoiding sample pollution and exhibiting high separation efficiency. Hence, this method is suitable for use in purifying organic material for OLEDs.

A currently useful method of purifying organic material for OLEDs is a train sublimation method. This method is performed in a manner whereby the material to be purified is placed in one end of a long hollow pipe and the pipe is heated using a heater under the condition that the inside of the pipe is evacuated using a vacuum pump, thus forming a temperature gradient throughout the pipe. Thereby, the material may be separated using the difference in recrystallization position, due to different sublimation points between the material to be separated and the impurities. In some situations, nitrogen or an inert gas that does not react with the material used for a purification apparatus is allowed to flow within a range such that the vacuum level is not greatly decreased from a high temperature to a low temperature, and may thus be used as a carrier gas for delivering the gas of the organic material. Such a carrier gas enables the efficient flow of the gas of the organic material.

FIG. 2 schematically illustrates the configuration of a conventional sublimation purification apparatus for performing a train sublimation method. As illustrated in FIG. 2, a raw organic material is placed in a crucible 240, and the crucible 240 is disposed at one side of a second quartz glass tube 210. The outer surface of the second quartz glass tube 210 is enclosed with a first quartz glass tube 220. As such, the crucible 240 is fitted into both open ends of a hollow cylindrical quartz tube, which is not shown, and the crucible is made of stainless steel and has a pair of lids having holes.

A heater 250 is provided around one side of the first quartz glass tube 220. As such, the heater 250 is disposed at a position corresponding to the position of the crucible 240. A vacuum pump 230 is disposed at the other side of the second quartz glass tube 210 so that the inside of the second quartz glass tube 210 is maintained in a vacuum.

In the sublimation purification apparatus 200 thus configured, the inside of the second quartz glass tube 210 is evacuated using the vacuum pump 230, and a small amount of carrier gas is allowed to flow throughout the second quartz glass tube 210 equipped with the vacuum pump 230 to form a fine pressure gradient. When the temperature is gradually increased using the heater 250, a temperature gradient is formed throughout the second quartz glass tube 210, and the temperature distribution thus formed shows a normal distribution curve.

When the temperature of the crucible 240 is higher than the sublimation point of the raw organic material to be purified therein, the material begins to be sublimated. As such, the resulting gas molecule is discharged from the crucible 240 and then begins to be moved toward the vacuum pump 230 by the pressure gradient. Impurities having a higher sublimation point than that of the raw organic material are left behind in the crucible 240.

The gas molecule that moves toward the vacuum pump 230 is transferred again into a solid phase in the zone of the second quartz glass tube 210, which has a temperature equal to or less than the sublimation point, and is then formed in a crystalline phase on the inner surface of the second quartz glass tube 210. Reference numeral 260 designates the purified material formed in a crystalline phase on the inner surface of the second quartz glass tube 210. After the lapse of a predetermined period of time, heating is stopped and gradual cooling occurs, so that the temperature of the second quartz glass tube equals room temperature. Then, the second quartz glass tube 210 is separated, and the purified material 260 in a crystalline phase is recovered by scraping.

However, the organic material (purified material) for OLEDs has to exhibit high purity with a very small amount of impurities, and thus it is difficult to obtain a material having desired purity through only a single purification process. Thus, since the purification method using the apparatus of FIG. 2 is performed in such a way that the sublimation purification process is repeated several times to obtain a highly pure material, the work time required to repeat the purification process may be considerably lengthened. Hence, this method is unsuitable for use in mass production.

Furthermore, the sublimation purification process is implemented in multiple steps, and thus the organic material is inevitably purified in an amount less than 70% of the initially introduced amount, undesirably causing high material cost and high power consumption.

In the sublimation method, the raw material may be purified to attain an organic material having high purity using the sublimation point difference of organic materials. However, while the purification process is performed in such a way that sublimation and condensation are repeated, a considerable amount of organic material may be lost together with the inert gas, undesirably resulting in very low yield of the final purified material relative to the amount of starting material. Furthermore, the time and cost required to optimize the purification system are high in consideration of the type of organic material.

With the goal of solving such problems, the present inventors have proposed that purification processing factors based on ionic liquid processing using an ionic liquid usable in a vacuum as a solvent may be rapidly optimized by appropriately selecting the purification temperature of the organic material, optimal ionic liquid, etc., thus ensuring a novel process of purifying an organic material using an ionic liquid process, which enables the mass purification of organic material for OLEDs.

However, since the organic material for OLEDs is very expensive, a problem of high processing cost may occur when testing the mass purification process using an ionic liquid process. Hence, there is a need for a simplified purification system for searching the availability of a novel purification process and optimizing the processing conditions by quickly verifying the recrystallization of a sublimated gas of the organic material to be purified using a variety of ionic liquids on a lab scale and by measuring the temperature of the ionic liquid for such recrystallization, along with changes in solubility depending on pressure and temperature. Thereby, a reduction in the processing cost of the novel mass purification process using an ionic liquid process, a shortened processing time for process optimization, etc., can be expected.

### Disclosure

### Technical Problem

Accordingly, the present invention has been made keeping in mind the above problems encountered in the related art, and an object of the present invention is to provide a simplified method and apparatus for purifying an organic material using an ionic liquid, wherein the process of purifying an organic material may be experimentally verified by recrystallizing a small amount of organic material using an ionic liquid that is stable in a vacuum.

Another object of the present invention is to provide a method and apparatus for purifying an organic material using an ionic liquid, wherein a large amount of organic material for OLEDs may be easily purified using, as a filter, an ionic liquid that is stable in a vacuum.

### Technical Solution

In order to accomplish the above objects, the present invention provides a simplified method of purifying an organic material using an ionic liquid, which uses a simplified apparatus for purifying an organic material comprising a heating chamber in a vacuum atmosphere including a crucible for receiving a raw organic material containing impurities for OLEDs, and a substrate disposed in the heating chamber and coated with an ionic liquid, the simplified method comprising: heating the crucible to a sublimation point of the raw organic material, thus generating a sublimated gas of the raw organic material; depositing the sublimated gas of the raw organic material on the ionic liquid; and recrystallizing the sublimated gas in the ionic liquid.

Also, according to the present invention, the simplified method may further comprise separating the recrystallized organic material from the ionic liquid, after the recrystallizing.

Also, according to the present invention, in the recrystallizing, a temperature of the ionic liquid may be adjusted to control a solubility of the ionic liquid.

Also, according to the present invention, in the recrystallizing, a temperature of the substrate may be maintained in a range from room temperature to 200°C.

In addition, the present invention provides a simplified apparatus for purifying an organic material using an ionic liquid, comprising: a sublimation unit in a vacuum atmosphere for heating a raw organic material containing impurities for OLEDs so as to be sublimated; a recrystallization unit in a vacuum atmosphere for depositing a sublimated gas of the organic material on a surface of the ionic liquid so as to be recrystallized in the ionic liquid; and a control unit for controlling operation of the sublimation unit and the recrystallization unit.

Also, according to the present invention, the sublimation unit may comprise a crucible for receiving the raw organic material, a heating chamber in which the crucible is disposed and which has a predetermined inner volume, a vacuum pump for evacuating an inside of the heating chamber, a first heater for heating the crucible, and a shutter for selectively opening or closing a top of the crucible.

Also, according to the present invention, the shutter may be provided to cover the top of the crucible, or may be formed to cover the top of the crucible at a predetermined distance therefrom.

Also, according to the present invention, the recrystallization unit may comprise a substrate coated with the ionic liquid, a mask for supporting the substrate, a second heater fixed to an upper portion of the heating chamber, and a support member formed under the second heater to support the mask.

Also, according to the present invention, the recrystallization unit may further comprise a thermocouple provided to the support member to measure a temperature of the substrate, and the control unit may control a temperature of the second heater using the temperature measured by the thermocouple.

Also, according to the present invention, the ionic liquid may be applied in droplet form on the substrate.

Also, according to the present invention, the simplified apparatus may further comprise an analysis unit for analyzing a purified material recrystallized in the ionic liquid via photography.

Also, according to the present invention, the analysis unit may comprise a thickness monitor for measuring a thickness of the recrystallized purified material.

In addition, the present invention provides a method of purifying an organic material using an ionic liquid, which uses an apparatus for purifying an organic material comprising a heating chamber in a vacuum atmosphere including a crucible for receiving a raw organic material containing impurities for OLEDs, and a storage tank containing the ionic liquid in a vacuum atmosphere, the method comprising: heating the crucible to a sublimation point of the raw organic material, thus generating a sublimated gas of the raw organic material; inducing the sublimated gas of the raw organic material to flow into the ionic liquid of the storage tank; and dissolving and recrystallizing, in the ionic liquid, the sublimated gas incorporated into the ionic liquid.

Also, according to the present invention, in the inducing the sublimated gas of the raw organic material to flow, the sublimated gas is induced to flow into the ionic liquid using an inert gas that is supplied into the heating chamber.

Also, according to the present invention, the method may further comprise discharging from the storage tank the inert gas that was not dissolved but was collected in an upper portion of the storage tank, after the dissolving.

Also, according to the present invention, the method may further comprise recovering the recrystallized organic material from the ionic liquid, after the recrystallizing.

Also, according to the present invention, the method may further comprise heating a gas mixture so as to maintain a temperature equal to or higher than the sublimation point, before incorporating the gas mixture into the ionic liquid.

Also, according to the present invention, in the recrystallizing, a temperature of the ionic liquid may be adjusted to control a solubility of the ionic liquid.

Also, according to the present invention, the inert gas discharged in the discharging may be reused as the inert gas that is supplied in mixing.

In addition, the present invention provides an apparatus for purifying an organic material using an ionic liquid, comprising: a sublimation unit in a vacuum atmosphere for heating a raw organic material containing impurities for OLEDs so as to be sublimated; a recrystallization unit in a vacuum atmosphere for incorporating a sublimated gas of the organic material into the ionic liquid so as to be recrystallized in the ionic liquid; and a control unit for controlling operation of the sublimation unit and the recrystallization unit.

Also, according to the present invention, the sublimation unit may comprise a crucible for receiving the raw organic material, a heating chamber in which the crucible is disposed and which has a predetermined inner volume, a vacuum pump for evacuating an inside of the heating chamber, a first heater for heating the crucible, and an inert gas supply source connected to one side of the heating chamber to supply the inert gas.

Also, according to the present invention, the recrystallization unit may comprise a storage tank containing the ionic liquid, a connection conduit, one side of which communicates with an inside of the heating chamber and the other side of which is immersed in the ionic liquid of the storage tank, a vacuum pump for evacuating an inside of the storage tank, and a discharge pump for discharging from the storage tank the gas accumulated on the ionic liquid of the storage tank.

Also, according to the present invention, the apparatus may further comprise a second heater for heating a periphery of the connection conduit so as to maintain a sublimation point of the organic material.

Also, according to the present invention, the apparatus may further comprise a third heater for heating a lower portion of the storage tank containing the ionic liquid to control a solubility of the organic material.

Also, according to the present invention, the apparatus may further comprise an ionic liquid collector provided at an upper portion of the storage tank so that the ionic liquid used for purifying the organic material is collected so as to be reused through heating to a predetermined temperature or higher in a vacuum, evaporation, and separation from the impurities and the dissolved organic material.

Also, according to the present invention, the ionic liquid collector may comprise a collection sheet fixed to an inner surface of the storage tank, and a collection tub fixed to an inner surface of the storage tank to gather the ionic liquid collected by the collection sheet.

Also, according to the present invention, the apparatus may further comprise an ionic liquid returning unit for returning the ionic liquid collected in the collection tub to the storage tank to reuse the ionic liquid.

Also, according to the present invention, the apparatus may further comprise a recovery tub that selectively communicates with the storage tank and is removably attached to the storage tank to separately recover the recrystallized organic material.

Also, according to the present invention, the apparatus may further comprise a bubble fining unit for reducing a volume of bubbles produced by incorporating a gas mixture into the ionic liquid of the storage tank.

Also, according to the present invention, the apparatus may further comprise an inert gas returning unit for returning the inert gas discharged by the discharge pump to the inert gas supply source to reuse the inert gas.

### Advantageous Effects

According to the present invention, even when a very small amount of organic material is sublimated, recrystallization thereof in an ionic liquid can be verified, and thus the purification potential of the organic material can be easily checked using only a very small amount of organic material.

Also, according to the present invention, the sublimated gas of the organic material can be easily supersaturated in a very small amount of ionic liquid due to the non-volatility of the ionic liquid, and thus the recrystallization of the organic material can be quickly verified.

Also, according to the present invention, the apparatus can be simply configured, and thus even when a different organic material is used, the cost and time required to search the optimal ionic liquid and to optimize the processing conditions can be reduced.

Also, according to the present invention, the sublimated gas generated upon sublimating the organic material can be incorporated into an ionic liquid that is stable even in a vacuum, and thus the ionic liquid can be utilized as a liquid filter for the sublimated gas, thereby greatly increasing the purification yield of the organic material.

Also, according to the present invention, the capacity of the apparatus can be adjusted, and thus, as a large amount of ionic liquid is used, the organic material can be added till supersaturation into the ionic liquid, ultimately enabling mass purification of the organic material, thereby reducing the cost of the organic material.

Moreover, since the ionic liquid can be reused via a repurification process, the manufacturing process can become environmentally friendly.

### Description of Drawings

FIG. 1 illustrates the structure of an OLED;
FIG. 2 schematically illustrates the configuration of a conventional sublimation purification apparatus;
FIG. 3 illustrates the configuration of a simplified apparatus for purifying an organic material using an ionic liquid according to a first embodiment of the present invention;
FIG. 4 is a flowchart illustrating a purification process, which uses the simplified apparatus for purifying an organic material using an ionic liquid as illustrated in FIG. 3;
FIG. 5 illustrates the configuration of an apparatus for purifying an organic material using an ionic liquid according to a second embodiment of the present invention;
FIG. 6 is a flowchart illustrating a purification process, which uses the apparatus for purifying an organic material using an ionic liquid as illustrated in FIG. 5;
FIG. 7 illustrates the configuration of an apparatus for purifying an organic material using an ionic liquid according to a third embodiment of the present invention;
FIG. 8 is a perspective view schematically illustrating the configuration of a simplified organic material purification tester using an ionic liquid;
FIG. 9 is a graph illustrating changes in the substrate temperature depending on the set temperature of a ceramic heater in the simplified organic material purification tester of FIG. 8;
FIGS. 10 to 12 are graphs illustrating changes in the deposition rate and the total thickness of the organic material depending on the sublimation temperature of the organic material at different crystallization temperatures (substrate temperatures) in the simplified organic material purification tester of FIG. 8;
FIG. 13 illustrates optical microscope images of the procedure of crystallizing an NPB organic material in an ionic liquid depending on the evaporation temperature of the organic material and the substrate temperature in the simplified organic material purification tester of FIG. 8;
FIG. 14 illustrates images (SEM analysis) of the crystallinity of the NPB organic material obtained using the simplified organic material purification tester of FIG. 8;
FIG. 15 is a graph illustrating the results of Raman analysis of the crystalline phase of the NPB organic material recrystallized using the simplified organic material purification tester of FIG. 8 and the crystalline phase of the NPB organic material before purification; and
FIG. 16 illustrates optical microscope images of the grain size of the NPB organic material recrystallized using the simplified organic material purification tester of FIG. 8 and the grain size of the NPB organic material before purification.

### Best Mode

Hereinafter, a detailed description will be given of a method and apparatus for purifying an organic material using an ionic liquid according to embodiments of the present invention with reference to the appended drawings.

### <First Embodiment>

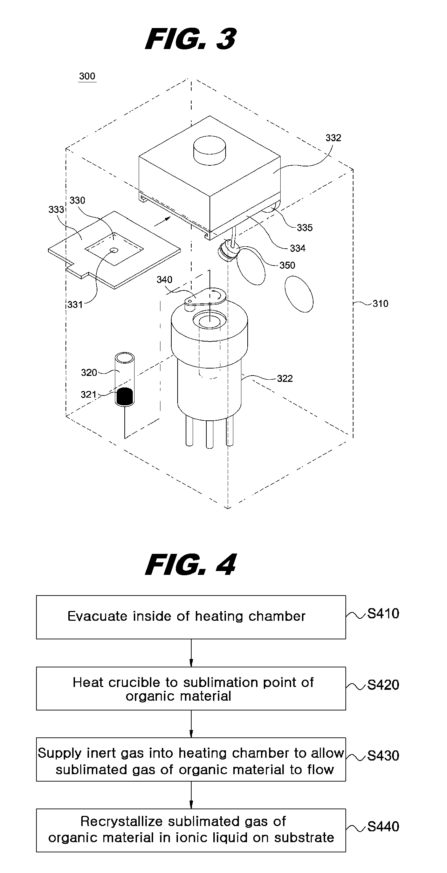

FIG. 3 is a perspective view illustrating the configuration of a simplified apparatus for purifying an organic material using an ionic liquid according to a first embodiment of the present invention. As illustrated in FIG. 3, the simplified apparatus 300 for purifying an organic material according to the present embodiment comprises a sublimation unit for sublimating a raw organic material, a recrystallization unit for recrystallizing the sublimated gas of the organic material in an ionic liquid, an analysis unit for analyzing the purified material recrystallized in the ionic liquid via photography, and a control unit for controlling the overall operation of the sublimation unit, the recrystallization unit, and the analysis unit.

According to the present embodiment, the ionic liquid may include 1-butyl-3-methylimidazolium bis(trifluoromethylsulfonyl)imide (BMIM TFSI) of Chemical Formula 1 below, or 1-octyl-3-methylimidazolium bis(trifluoromethylsulfonyl)imide (OMIM TFSI) of Chemical Formula 2 below. Alternatively useful is 1-etyl-3-methylimidazolium bis(trifluoromethylsulfonyl)imide (EMIM TFSI).

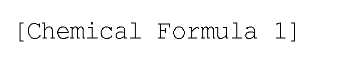

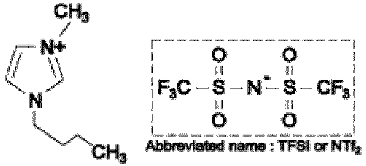

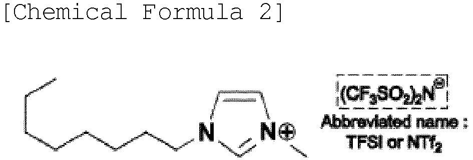

The aforementioned ionic liquids (BMIM TFSI, OMIM TFSI, EMIM TFSI) are non-volatile organic solvents, and may be used to purify and recrystallize various organic materials via a mechanism wherein, in the course of repeated dissolution and recrystallization of the organic material and the impurities in the ionic liquid, the organic material that most quickly reaches supersaturation is recrystallized first.

Also, BMIM TFSI, OMIM TFSI, and EMIM TFSI have low melting points, low vapor pressure, and nonflammability, are composed of organic molecular ions, and have properties that are controllable by combinations of anions and cations.

According to the present embodiment, the ionic liquid is used to purify and recrystallize the organic material, and is stable in a liquid phase even under conditions of 100 to 120°C and 10⁻⁷ Torr, and thus may be utilized as a solvent in a vacuum process.

According to the present embodiment, the raw organic material may be exemplified by NPB (N,N'-bis(naphthalen-1-yl)-N,N'-bis(phenyl)-benzidine), which is useful as a material for a hole transport layer (HTL). NPB has a sublimation point of 180°C or more. Thus, when the crucible in the heating chamber for the sublimation unit is heated to 200°C or more, sublimation occurs.

Also, examples of a deposition material (a raw organic material) for use in manufacturing an OLED device may include a variety of materials in addition to the above material. Thus, various kinds of organic materials may be employed as the raw material in the present invention.

The configuration of the constituents of the simplified apparatus 300 for purifying an organic material according to the present embodiment is specified below.

The sublimation unit comprises a crucible 320 for receiving a raw organic material 321 containing impurities, a heating chamber 310 in which the crucible 320 is disposed and which has a predetermined inner volume, a vacuum pump (not shown) for evacuating the inside of the heating chamber 310, a first heater 322 for heating the crucible 320, and a shutter 340 for selectively opening or closing the top of the crucible 320.

Although the shutter 340 may be provided to cover the top of the crucible 320, it is preferably formed to cover the top of the crucible 320 at a predetermined distance therefrom. Such a shutter 340 functions such that all the sublimated gas of the organic material to be sublimated by heating the crucible 320 may be moved toward the recrystallization unit.

The recrystallization unit plays a role in recrystallizing, in the ionic liquid, the sublimated gas of the organic material, and comprises a silicon substrate 330 coated with an ionic liquid 331, a mask 333 for supporting the silicon substrate 330, a second heater 332 fixed to the upper portion of the heating chamber 310, a support member 334 formed under the second heater 332 to support the mask 333, and a thermocouple 335 provided to the support member 334 to measure the temperature of the silicon substrate 330.

The ionic liquid 331 is applied in the form of a droplet on the silicon substrate 330. The thermocouple 335 functions to measure the actual temperature of the silicon substrate 330, and the actual temperature of the silicon substrate 330 depending on the temperature applied to the second heater 332 is used to adjust the temperature of the second heater 332 by the control unit.

The analysis unit plays a role in analyzing the purified material recrystallized in the ionic liquid 331 via photography, and may include a thickness monitor 350.

The control unit is responsible for overall controlling the temperature of the first heater 322 and the operation of the shutter 340 in the sublimation unit; the temperature of the second heater 332 of the recrystallization unit; and the operation of the analysis unit.

In addition, a method of purifying an organic material using the simplified apparatus for purifying an organic material according to the present embodiment is described below.

FIG. 4 is a flowchart illustrating a purification process, which uses the simplified apparatus for purifying an organic material using an ionic liquid as illustrated in FIG. 3. As illustrated in FIGS. 3 and 4, the crucible 320 containing the raw organic material 321 is disposed in the heating chamber 310 under the condition that the top of the crucible 320 is closed by the shutter 340, and the inside of the heating chamber 310 is evacuated using the vacuum pump (S410).

Next, the crucible 320 is heated to the sublimation point of the organic material using the first heater 322 (S420). Thereby, a sublimated gas of the organic material comprising the organic material and some impurities, which are mixed together, is formed. The sublimated gas of the organic material is gradually gathered by the shutter 340 that closes the top of the crucible 320.

After the lapse of a predetermined period of time, when the shutter is opened, the sublimated gas of the organic material is moved toward the silicon substrate 330 by its own driving force, so that the sublimated gas of the organic material is deposited on the ionic liquid 331 (S430). Next, the sublimated gas of the organic material comes into contact with the ionic liquid 331 applied on the silicon substrate 330, and is thus dissolved in the ionic liquid 331 and recrystallized (S440). When the sublimated gas of the organic material dissolved in the ionic liquid 331 is supersaturated, it is recrystallized and thus precipitated as a purified material having high purity.

As such, the temperature of the silicon substrate 330 is preferably maintained in the range from room temperature to 200°C. This is because the ionic liquid is present in ionic form due to the composition of a polymer material comprising C, H, F, N, and O elements, whereby the molecular structure thereof is broken at high temperatures and the inherent properties thereof are not maintained. Although the properties of the ionic liquid may vary slightly depending on the type thereof, they change at about 200°C, and thus the available temperature of the ionic liquid is limited to the range from room temperature to 200°C. In order to supersaturate the organic material within the range in which the properties of the ionic liquid do not change, the temperature of the ionic liquid is increased through the silicon substrate 330. If the temperature of the ionic liquid is lower than room temperature, a sufficient amount of organic material cannot be dissolved, making it impossible to recrystallize the organic material.

Meanwhile, information for the purified material precipitated on the ionic liquid 331 may be checked using the thickness monitor 350. Also, the precipitated purified material having high purity may be appropriately recovered from the heating chamber 310.

Therefore, even when a very small amount of organic material is sublimated, its dissolution and recrystallization in the ionic liquid 331 may be easily tested, and thus this invention may be utilized for a large-scale apparatus for purifying an organic material using an ionic liquid.

### <Second Embodiment>

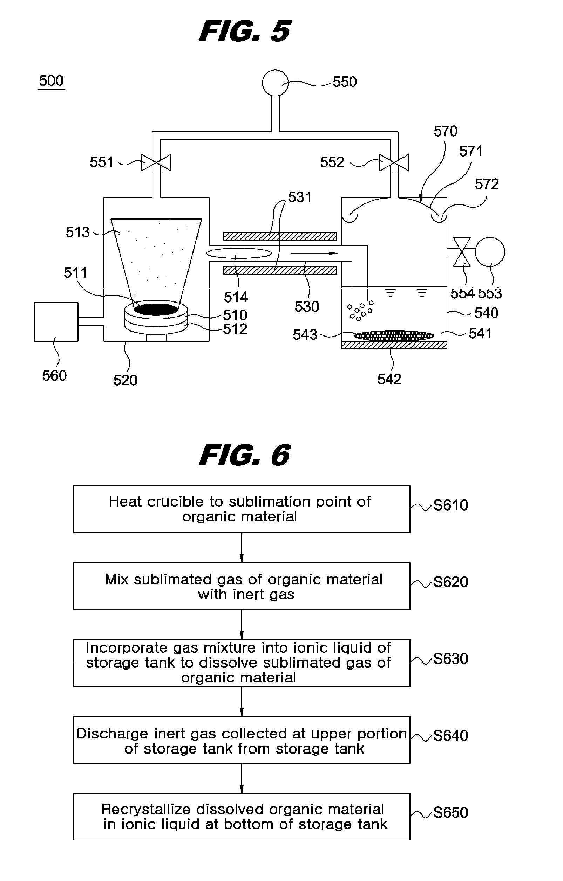

FIG. 5 illustrates the configuration of an apparatus for purifying an organic material using an ionic liquid according to a second embodiment of the present invention. As illustrated in FIG. 5, the apparatus 500 for purifying an organic material according to the present embodiment comprises a sublimation unit in a vacuum atmosphere for sublimating a raw organic material, containing impurities, for OLEDs by heating, a recrystallization unit in a vacuum atmosphere for incorporating the sublimated gas of the organic material into the ionic liquid so that such a gas is recrystallized in the ionic liquid, and a control unit for controlling the operation of the sublimation unit and the recrystallization unit.

The sublimation unit comprises a crucible 510 for receiving a raw organic material 511, a heating chamber 520 in which the crucible 510 is disposed and which has a predetermined inner volume, a vacuum pump 550 for evacuating the inside of the heating chamber 520, a first heater 512 for heating the crucible 510, and an inert gas supply source 560 connected to one side of the heating chamber 520 to supply an inert gas.

The recrystallization unit comprises a storage tank 540 containing the ionic liquid 541, a connection conduit 530, one side of which communicates with the inside of the heating chamber 520 and the other side of which is immersed in the ionic liquid 541 of the storage tank 540, the vacuum pump 550 for evacuating the inside of the storage tank 540, and a discharge pump 553 for discharging from the storage tank 540 the gas accumulated on the ionic liquid 541 of the storage tank 540.

Also, the heating chamber 520 and the storage tank 540 are connected to each other at the tops thereof, and the connection portion therebetween is provided with the vacuum pump 550. The connection line of the vacuum pump 550 is provided with respective valves 551, 552, which selectively communicate with the heating chamber 520 and the storage tank 540.

Also provided at the upper portion of the storage tank 540 may be an ionic liquid collector 570 for collecting the ionic liquid 541 used for purifying the organic material so that it can be reused through purification.

The ionic liquid 541 and the raw organic material 511 according to this embodiment are the same as those in the first embodiment.

The crucible 510 is disposed at the bottom of the heating chamber 520, and a first heater 512 is provided under the crucible. The crucible 510 is configured such that the raw organic material 511 to be purified may be contained therein.

One side of the connection conduit 530 is connected to the upper portion of the heating chamber 520, and the other side thereof is disposed to extend through the upper portion of the storage tank 540 so as to be immersed in the ionic liquid 541. A second heater 531 for heating the connection conduit 530 may be further provided around the connection conduit 530. This second heater 531 functions to heat the periphery of the connection conduit 530 so as to maintain the sublimation point of the sublimated gas mixture 513 in the course of incorporating the gas mixture into the ionic liquid 541 through the connection conduit 530, as will be described later.

Also, a third heater 542 may be provided at the lower end of the storage tank 540. The third heater 542 functions to heat the ionic liquid 541 so as to adjust the solubility of the sublimated gas mixture 513 dissolved in the ionic liquid 541. Also, a discharge pump 553 may be provided at the upper portion of the storage tank 540. The connection line of the discharge pump 553 is preferably further provided with a valve 554.

Also, an ionic liquid collector 570 may be provided at the upper portion of the storage tank 540. The ionic liquid collector 570 functions to collect the ionic liquid 541 used for purifying the organic material so that such an ionic liquid is reused through evaporation and separation from the dissolved organic material and the impurities. The ionic liquid collector comprises a collection sheet 571, which is fixed to the inner surface of the storage tank 540 and has a curved surface, and a collection tub 572, which is fixed to the inner surface of the storage tank 540 to gather the ionic liquid collected by the collection sheet 571.

In addition, a method of purifying an organic material using the apparatus for purifying an organic material according to the present embodiment is described below.

FIG. 6 is a flowchart illustrating a purification process, which uses the apparatus for purifying an organic material using an ionic liquid as shown in FIG. 5. As illustrated in FIGS. 5 and 6, the crucible 510 containing the raw organic material 511 is disposed in the heating chamber 520, and an appropriate amount of the ionic liquid 541 is placed in the storage tank 540, after which the heating chamber 520 and the storage tank 540 are evacuated using the vacuum pump 550. Subsequently, the crucible 510 is heated to the sublimation point of the organic material using the first heater 512. Thereby, the sublimated gas mixture 513 of the organic material comprising the organic material and some impurities, which are mixed together, is formed (S610).

Next, an inert gas is supplied into the heating chamber 520 from the inert gas supply source 560. The inert gas may include nitrogen or argon gas, which does not react with the material used for the apparatus 500 for purifying an organic material within the range in which the vacuum level does not greatly decrease. The inert gas functions to allow the sublimated gas mixture 513 to flow into the ionic liquid 541 of the storage tank 540, and is mixed with the sublimated gas mixture 513, thus forming a gas mixture (S620).

The gas mixture thus formed is incorporated into the ionic liquid 541 through the connection conduit 530 with an increase in the pressure inside the heating chamber 520, thus forming bubbles (S630). On the other hand, in the course of incorporating the gas mixture into the ionic liquid 541 through the connection conduit 530, as the periphery of the connection conduit 530 is heated by the second heater 531, which is provided around the connection conduit 530, the sublimated gas mixture 513 may be incorporated into the ionic liquid 541 while maintaining the sublimation point thereof.

While bubbles are formed by the gas mixture incorporated into the ionic liquid 541, the sublimated gas mixture 513 in the bubbles is dissolved in the ionic liquid 541, whereas the inert gas is not dissolved in the ionic liquid 541, but floats out of the ionic liquid 541 and is thereby collected in the upper portion of the storage tank 540. The inert gas collected in the upper portion of the storage tank 540 is discharged from the storage tank 540 by the discharge pump 553, and is then recovered (S640). The inert gas, which is recovered after having been discharged from the storage tank 540, is returned to the inert gas supply source 560 via an inert gas returning unit, and may thus be reused. As such, the inert gas returning unit may include a typical pump.

Since the amount of organic material to be purified relative to the amount of impurities is extremely high when dissolving the sublimated gas mixture 513 in the ionic liquid 541, the organic material first reaches supersaturation and then begins to be recrystallized, and is thus precipitated as a purified material 543 having high purity (S650). As such, the solubility of the sublimated gas mixture 513 dissolved in the ionic liquid 541 may be adjusted using the third heater 542 provided at the lower end of the storage tank 540. Thereby, the solubility of the ionic liquid 541 for the sublimated gas mixture 513 is regulated, making it possible to control the supersaturation of the organic material in the ionic liquid 541 and the rate of recrystallization of the organic material. Accordingly, the incorporation of impurities may be minimized during recrystallization, and the purified material 543 having high purity, which is precipitated in the ionic liquid 541, may be appropriately recovered from the heating chamber 520. For example, the purified material 543 may be recovered through an opening/closing port formed in one side of the storage tank 540.

When the purified material 543 having high purity, which is precipitated in the ionic liquid 541, is recovered in this way, some of the organic material, which is dissolved until it is supersaturated in the gas mixture, is left behind in the ionic liquid 541, along with a small amount of impurities. As the purification process proceeds, the amount of impurities in the ionic liquid 541 is increased, and may then reach supersaturation at a certain time point, thus incorporating impurities into the recrystallized organic material. At this time, the ionic liquid for the purification process is preferably exchanged with a highly pure ionic liquid.

The dissolved organic material and the impurities have different evaporation temperatures than the ionic liquid. Briefly, the evaporation temperature of the ionic liquid is lower than those of the organic material and the impurities. Hence, using this temperature difference, the ionic liquid component may be separately purified. To this end, when heating is performed under the condition that the temperature of the third heater 542 is set to the evaporation temperature of the ionic liquid, the ionic liquid is evaporated and recovered in the collection tub 572 through the collection sheet 571 of the ionic liquid collector 570, and the highly concentrated organic material and the impurities are left behind. The highly concentrated organic material and the impurities, which are left behind, are additionally collected, and are then treated again using a typical solvent to separate the organic material from the impurities, after which organic material having a predetermined purity may be reused as the feed for recrystallization. Also, the ionic liquid recovered in the collection tub 572 may be returned into the storage tank 540 using the ionic liquid returning unit and then reused. Herein, the ionic liquid returning unit may include a typical pump.

The apparatus 500 for purifying an organic material according to this embodiment may further comprise a bubble fining unit for decreasing the volume of bubbles so as to facilitate the dissolution of the sublimated gas mixture 513 of the bubbles in the ionic liquid 541 via contact after the gas mixture has been incorporated into the ionic liquid 541 of the storage tank 540.

Also, the apparatus 500 for purifying an organic material according to this embodiment may further comprise a contact enhancement unit for facilitating the contact of the sublimated gas mixture 513 with the ionic liquid 541. For example, the sublimated gas mixture 513 mixed with the inert gas is induced to pass through the ionic liquid 541 for a predetermined period of time, thereby promoting the dissolution of the sublimated gas.

### <Third Embodiment>

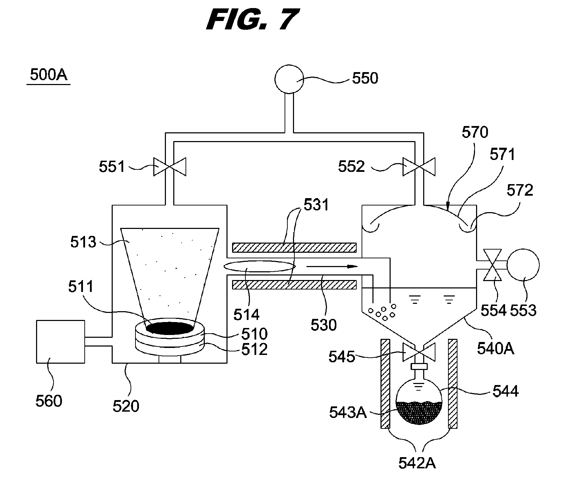

FIG. 7 illustrates the configuration of an apparatus for purifying an organic material using an ionic liquid according to a third embodiment of the present invention. As illustrated in FIG. 7, the apparatus 500A for purifying an organic material according to this embodiment has the same configuration as the apparatus 500 for purifying an organic material according to the second embodiment, with the exception that the shape of the storage tank 540A and the position of the third heater 542A are partially changed so as to enable efficient recovery of the purified material 543A. Hence, the same constituents in this embodiment are denoted with the same reference numerals, and a description thereof is omitted.

The storage tank 540A according to this embodiment is configured such that the lower portion thereof is provided in the form of a funnel, and a recovery tub 544 for recovering the purified material 543A is additionally provided at the lower end of the storage tank 540A. The recovery tub 544 is removably attached to the lower end of the storage tank 540A. Thus, the purified material 543A precipitated by the aforementioned procedure is gradually accumulated in the recovery tub 544. Also, a valve 545 for controlling the movement of the ionic liquid into the recovery tub 544 is further provided at the lower portion of the storage tank 540A. When a predetermined amount of the purified material 543A has accumulated in the recovery tub 544, the valve 545 is closed, and the recovery tub 544 is separated from the storage tank 540A, whereby the purified material 543A is recovered.

The third heater 542A according to this embodiment is provided at the side of the storage tank 540A because the recovery tub 544 is formed at the lower end of the storage tank 540A, and is thus responsible for indirectly heating the purified material 543A so as to be efficiently precipitated.

Test procedures and results for the purification effects including purification of the organic material using the ionic liquid as mentioned above are described below.

### 1. Simplified organic material purification tester

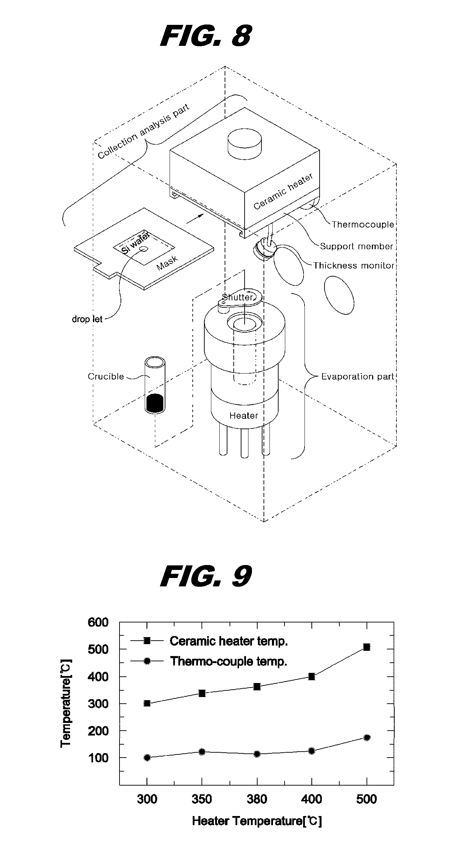

FIG. 8 is a perspective view schematically illustrating the configuration of a simplified organic material purification tester using an ionic liquid. As illustrated in FIG. 8, the simplified organic material purification tester comprises a sublimation part for sublimating a raw organic material, a recrystallization part for recrystallizing the sublimated organic material (purified material) in the ionic liquid, and an analysis part for analyzing the recrystallized organic material (purified material). The recrystallization part and the analysis part include a ceramic heater, a thickness monitor, a thermocouple, and a mask, and the sublimation part includes a shutter, a crucible, and a heater. In the recrystallization part, the ionic liquid is applied in the form of a droplet on a Si wafer, after which the Si wafer is fixed to the mask, and further fixed to a ceramic heater, and in the sublimation part, the raw organic material to be purified is loaded in the crucible.

Upon testing using the simplified organic material purification tester thus configured, the amount of the raw organic material in the sublimation part that evaporates was controlled by adjusting the temperature of the sublimation part, and the temperature of the ceramic heater was adjusted to crystallize, in the ionic liquid, the organic material supplied from the sublimation part, so as to determine the optimal crystallization temperature.

### 2. Preparation for organic material purification testing (sublimation part)

The raw organic material to be purified was weighed and then placed in the crucible, after which the crucible was seated in the sublimation part. The raw organic material that was used was NPB (N,N'-di(biphenyl-4-yl)-N,N'-bis(2-methylbiphenyl-4-yl)biphenyl-4,4'-diamine).

### 3. Preparation of organic material purification testing (recrystallization part and analysis part)

The ionic liquid was applied in the form of a droplet on the Si wafer (50x50mm²) and then the Si wafer was mounted to a mask. Subsequently, the mask was fixed to a support member connected to a ceramic heater, after which the crystallization temperature of the organic material and the amount of the organic material supplied from the sublimation part were measured using a thermocouple and a thickness monitor, thus determining the optimal organic material purification conditions. The ionic liquid that was used was OMIM TFSI.

### 4. Ceramic heater-thermocouple temperature

FIG. 9 is a graph illustrating changes in the substrate temperature depending on the set temperature of the ceramic heater in the simplified organic material purification tester of FIG. 8. As illustrated in FIG. 9, the set temperature of the ceramic heater provided to the tester was increased to 300 ∼ 500°C, and the temperature actually transferred to the recrystallization part was measured using a thermocouple, thus obtaining the temperature gradient shown in FIG. 9.

The test for changes in the temperature was performed to determine the crystallization temperature of the ionic liquid applied on the Si wafer. Based on the test results, the temperature measured by the thermocouple, not the temperature of the ceramic heater, was taken as the actual crystallization temperature, and the purification test was continued.

### 5. Changes in deposition rate depending on organic material sublimation temperature of sublimation part (crystallization temperature: R.T.)

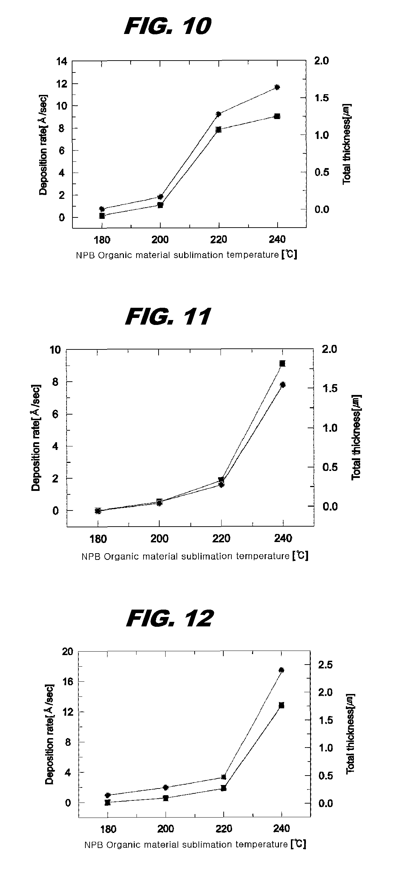

FIG. 10 is a graph illustrating changes in the deposition rate and the total thickness of the organic material depending on the sublimation temperature of the NPB organic material when the crystallization temperature (substrate temperature) is room temperature in the simplified organic material purification tester of FIG. 8. When the crystallization temperature was room temperature, the deposition rate and the total thickness of the NPB organic material depending on changes in the sublimation temperature of the sublimation part were analyzed using a thickness monitor. Based on the test results, as illustrated in FIG. 10, as the sublimation temperature of the organic material was raised, the deposition rate was increased to 0.1 to 9 Å/sec, and the total thickness of the organic material was changed to 0.011 to 1.64 µm.

### 6. Changes in deposition rate depending on organic material sublimation temperature of sublimation part (crystallization temperature: 100°C)

FIG. 11 is a graph illustrating changes in the deposition rate and the total thickness of the organic material depending on the sublimation temperature of the NPB organic material when the crystallization temperature (substrate temperature) is 100°C in the simplified organic material purification tester of FIG. 8. When the crystallization temperature was 100°C, the deposition rate and the total thickness of the NPB organic material depending on changes in the sublimation temperature of the sublimation part were analyzed using a thickness monitor. Based on the test results, as illustrated in FIG. 11, as the sublimation temperature of the organic material was raised, the deposition rate was increased to 0.1 to 9.2 Å/sec, and the total thickness of the organic material was changed to 0.011 to 1.51 µm.

### 7. Changes in deposition rate depending on organic material sublimation temperature of sublimation part (crystallization temperature: 110°C)

FIG. 12 is a graph illustrating changes in the deposition rate and the total thickness of the organic material depending on the sublimation temperature of the NPB organic material when the crystallization temperature (substrate temperature) is 110°C in the simplified organic material purification tester of FIG. 8. When the crystallization temperature was 110°C, the deposition rate and the total thickness of the NPB organic material depending on changes in the sublimation temperature of the sublimation part were analyzed using a thickness monitor. Based on the test results, as illustrated in FIG. 12, as the sublimation temperature of the organic material was raised, the deposition rate was increased to 0.1 to 12.8 Å/sec, and the total thickness of the organic material was changed to 0.013 to 2.37 µm.

### 8. Optical microscope images of crystallized NPB organic material in ionic liquid

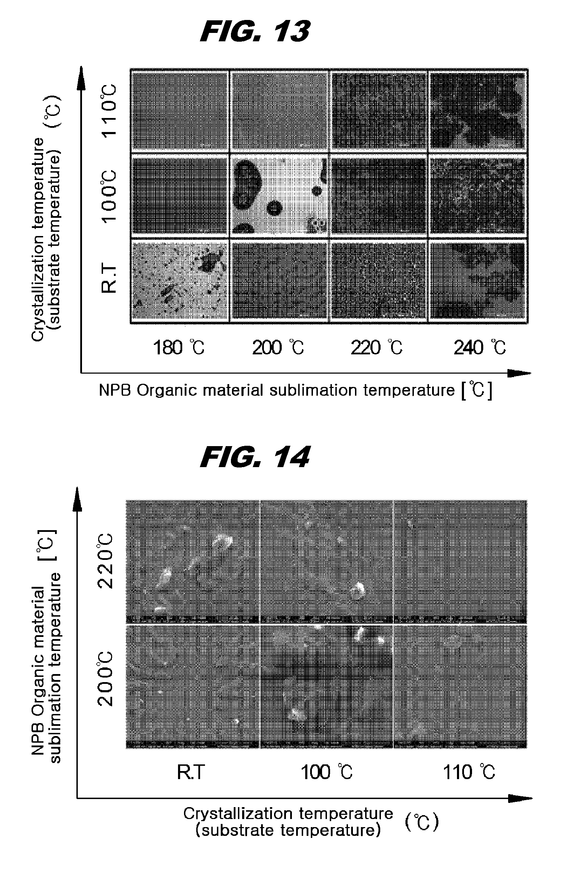

FIG. 13 illustrates optical microscope images of the crystallization procedure of the NPB organic material in the ionic liquid depending on the organic material sublimation temperature and the substrate temperature in the simplified organic material purification tester of FIG. 8. As illustrated in FIG. 13, sublimation of the NPB organic material did not occur at the sublimation temperature (180°C) of the sublimation part, and thus the material was not supplied to the ionic liquid. However, as the sublimation temperature of the NPB organic material was raised to 200°C or more, sublimation took place, and the grain size of the purified NPB organic material was enlarged with an increase in the crystallization temperature (substrate temperature) of the ionic liquid.

### 9. SEM images of crystallized NPB organic material in ionic liquid

FIG. 14 illustrates the images (SEM analysis) of crystallinity of the NPB organic material obtained using the simplified organic material purification tester of FIG. 8. Specifically, FIG. 14 illustrates the results of SEM surface analysis of the crystallinity of the NPB organic material depending on the organic material sublimation temperature (200, 220°C) and the crystallization temperature (R.T., 100, 110°C), based on the results of optical microscopy analysis of the crystallinity of the NPB organic material. As illustrated in FIG. 14, the crystallinity of the NPB organic material was highest when the crystallization temperature of the ionic liquid was R.T. or 100°C, and the crystallinity was decreased at the crystallization temperature (110°C).

### 10. RAMAN analysis of crystalline phase of NPB organic material

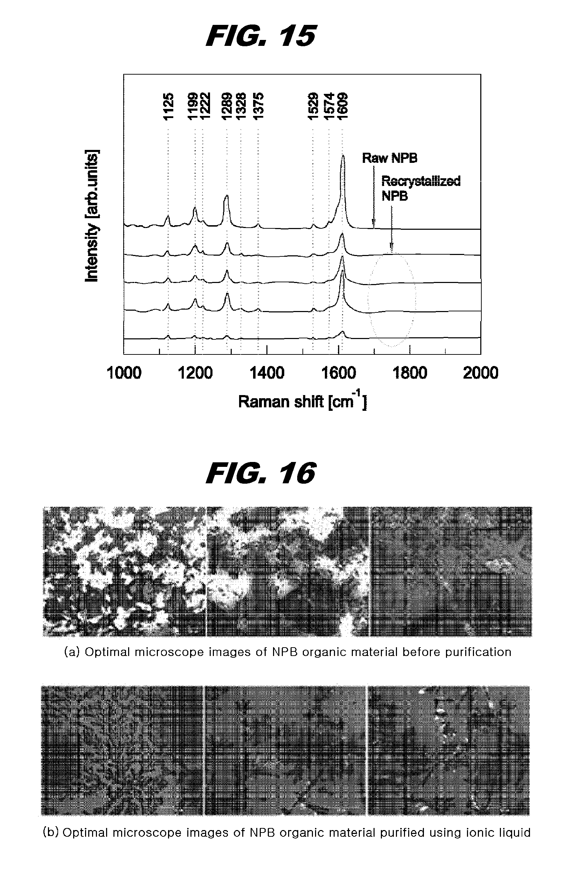

FIG. 15 is a graph illustrating the results of Raman analysis of the crystalline phase of the NPB organic material recrystallized using the simplified organic material purification tester of FIG. 8, and the crystalline phase of the NPB organic material before purification. As shown in FIG. 15, the crystal peaks of the NPB organic material recrystallized under purification conditions including the sublimation temperature (220°C) of the NPB organic material and the crystallization temperature (substrate temperature) (110°C) exhibited Raman shift values of 1125, 1199, 1222, 1289, 1328, 1375, 1529, 1574, and 1609 cm⁻¹, and these Raman shift values were almost the same as those of the crystalline phase of the NPB organic material before purification. This shows that, upon recrystallization using the ionic liquid, the raw NPB organic material was recrystallized while maintaining the original crystallinity thereof.

### 11. Optical microscopy analysis of grain size of NPB organic material

FIG. 16 illustrates optical microscope images of the grain size (b) of the NPB organic material recrystallized using the simplified organic material purification tester of FIG. 8 and the grain size (a) of the NPB organic material before purification. As illustrated in FIG. 16, based on the results of measurement of the grain shape and size of the NPB organic material before purification at different magnifications (x50, x100, x200), a random directional grain shape in a powder phase was manifested, and the grain size was various, without any particular pattern. However, the NPB organic material recrystallized in the ionic liquid exhibited a grain shape having a dendrite structure, and the diameter of the crystallized NPB organic material was a maximum of 50 µm, and thus the crystallinity of the NPB organic material was greatly improved compared to the raw material.

The crystalline phases of the raw material and the purified NPB organic material in Raman data were almost the same, making it difficult to accurately estimate the improvement in crystallinity. Through optical microscopy analysis, while the crystalline phase of the raw material was maintained, the crystallinity and the grain size could be improved using an ionic liquid purification method.

### 12. Conclusion

Based on the data analysis results of the NPB organic material purified using the ionic liquid and the raw material, there were obvious changes in the recrystallized NPB organic material. Compared to the sublimation method for purifying the organic material by repeating a plurality of sublimation and condensation procedures, the recrystallization method using the supersaturation of the organic material in the ionic liquid is advantageous in terms of simple processing and also increased material purity by recrystallizing only the pure organic material.

Although this test was conducted using a small simplified organic material purification tester, it can be applied both to a simplified organic material purification technique as in the present invention and to a mass organic material purification technique, as seen in the above test results.

### Industrial Applicability

As is apparent from the aforementioned examples and test examples, the present invention addresses a purification process using the solubility difference. Thus, a variety of organic materials can be purified and recrystallized through only a single process by a mechanism wherein, in the course of repeated dissolution and recrystallization of the organic material and the impurities in the ionic liquid, the organic material that most quickly reaches supersaturation is recrystallized first.

Also, according to the present invention, as the organic material is purified in a vacuum, the amount of impurities supplied from an external pollution source can be minimized, thereby obtaining a highly pure organic material (purified material).

Also, according to the present invention, since a purification process is carried out in an ionic liquid, which serves as a filter, there is no material loss attributable to carrier gas. Furthermore, the recrystallized organic material is recovered, after which the ionic liquid can be reused to purify the organic material, whereby the effects of reducing both the loss of raw materials consumed in the purification process and the manufacturing cost can be expected.

Although the preferred embodiments of the present invention regarding the method and apparatus for purifying the organic material using the ionic liquid have been disclosed with reference to the appended drawings, they are merely illustrative. Therefore, the present invention is not limited to such embodiments, and those skilled in the art will appreciate that various modifications and variations thereof are possible, without departing from the spirit and scope of the present invention, and also fall within the claims of the present invention.

## Claims

1. A simplified method of purifying an organic material using an ionic liquid, which uses a simplified apparatus for purifying an organic material comprising a heating chamber in a vacuum atmosphere including a crucible for receiving a raw organic material containing impurities for an organic light emitting diode (OLED), and a substrate disposed in the heating chamber and coated with an ionic liquid, the simplified method comprising:
heating the crucible to a sublimation point of the raw organic material, thus generating a sublimated gas of the raw organic material;
depositing the sublimated gas of the raw organic material on the ionic liquid; and
recrystallizing the sublimated gas in the ionic liquid.

2. The simplified method of claim 1, further comprising separating the recrystallized organic material from the ionic liquid, after the recrystallizing.

3. The simplified method of claim 1, wherein, in the recrystallizing, a temperature of the ionic liquid is adjusted to control a solubility of the ionic liquid.

4. The simplified method of claim 1, wherein, in the recrystallizing, a temperature of the substrate is maintained in a range from room temperature to 200°C.

5. A simplified apparatus for purifying an organic material using an ionic liquid, comprising:
a sublimation unit in a vacuum atmosphere for heating a raw organic material containing impurities for an organic light emitting diode (OLED) so as to be sublimated;
a recrystallization unit in a vacuum atmosphere for depositing a sublimated gas of the organic material on a surface of the ionic liquid so as to be recrystallized in the ionic liquid; and
a control unit for controlling operation of the sublimation unit and the recrystallization unit.

6. The simplified apparatus of claim 5, wherein the sublimation unit comprises a crucible for receiving the raw organic material, a heating chamber in which the crucible is disposed and which has a predetermined inner volume, a vacuum pump for evacuating an inside of the heating chamber, a first heater for heating the crucible, and a shutter for selectively opening or closing a top of the crucible.

7. The simplified apparatus of claim 6, wherein the shutter is provided to cover the top of the crucible, or is formed to cover the top of the crucible at a predetermined distance therefrom.

8. The simplified apparatus of claim 6, wherein the recrystallization unit comprises a substrate coated with the ionic liquid, a mask for supporting the substrate, a second heater fixed to an upper portion of the heating chamber, and a support member formed under the second heater to support the mask.

9. The simplified apparatus of claim 8, wherein the recrystallization unit further comprises a thermocouple provided to the support member to measure a temperature of the substrate, and
the control unit controls a temperature of the second heater using the temperature measured by the thermocouple.

10. The simplified apparatus of claim 5, wherein the ionic liquid is applied in droplet form on the substrate.

11. The simplified apparatus of any one of claims 5 to 10, further comprising an analysis unit for analyzing a purified material recrystallized in the ionic liquid via photography.

12. The simplified apparatus of claim 11, wherein the analysis unit comprises a thickness monitor for measuring a thickness of the recrystallized purified material.

13. A method of purifying an organic material using an ionic liquid, which uses an apparatus for purifying an organic material comprising a heating chamber in a vacuum atmosphere including a crucible for receiving a raw organic material containing impurities for an organic light emitting diode (OLED), and a storage tank containing the ionic liquid in a vacuum atmosphere, the method comprising:
heating the crucible to a sublimation point of the raw organic material, thus generating a sublimated gas of the raw organic material;
inducing the sublimated gas of the raw organic material to flow into the ionic liquid of the storage tank; and
dissolving and recrystallizing, in the ionic liquid, the sublimated gas incorporated into the ionic liquid.

14. The method of claim 13, wherein, in the inducing the sublimated gas of the raw organic material to flow, the sublimated gas is induced to flow into the ionic liquid using an inert gas that is supplied into the heating chamber.

15. The method of claim 14, further comprising discharging from the storage tank the inert gas that was not dissolved but was collected in an upper portion of the storage tank, after the dissolving.

16. The method of claim 15, further comprising recovering the recrystallized organic material from the ionic liquid, after the recrystallizing.

17. The method of claim 15, further comprising heating a gas mixture so as to maintain a temperature equal to or higher than the sublimation point, before incorporating the gas mixture into the ionic liquid.

18. The method of claim 15, wherein, in the recrystallizing, a temperature of the ionic liquid is adjusted to control a solubility of the ionic liquid.

19. The method of claim 15, wherein the inert gas discharged in the discharging is reused as the inert gas that is supplied in mixing.

20. An apparatus for purifying an organic material using an ionic liquid, comprising:
a sublimation unit in a vacuum atmosphere for heating a raw organic material containing impurities for an organic light emitting diode (OLED) so as to be sublimated;
a recrystallization unit in a vacuum atmosphere for incorporating a sublimated gas of the organic material into the ionic liquid so as to be recrystallized in the ionic liquid; and
a control unit for controlling operation of the sublimation unit and the recrystallization unit.

21. The apparatus of claim 20, wherein the sublimation unit comprises a crucible for receiving the raw organic material, a heating chamber in which the crucible is disposed and which has a predetermined inner volume, a vacuum pump for evacuating an inside of the heating chamber, a first heater for heating the crucible, and an inert gas supply source connected to one side of the heating chamber to supply the inert gas.

22. The apparatus of claim 21, wherein the recrystallization unit comprises a storage tank containing the ionic liquid, a connection conduit, one side of which communicates with an inside of the heating chamber and the other side of which is immersed in the ionic liquid of the storage tank, a vacuum pump for evacuating an inside of the storage tank, and a discharge pump for discharging from the storage tank the gas accumulated on the ionic liquid of the storage tank.

23. The apparatus of claim 22, further comprising a second heater for heating a periphery of the connection conduit so as to maintain a sublimation point of the organic material.

24. The apparatus of claim 22, further comprising a third heater for heating a lower portion of the storage tank containing the ionic liquid to control a solubility of the organic material.

25. The apparatus of claim 24, further comprising an ionic liquid collector provided at an upper portion of the storage tank so that the ionic liquid used for purifying the organic material is collected so as to be reused through heating to a predetermined temperature or higher in a vacuum, evaporation, and separation from the impurities and the dissolved organic material.

26. The apparatus of claim 25, wherein the ionic liquid collector comprises a collection sheet fixed to an inner surface of the storage tank, and a collection tub fixed to an inner surface of the storage tank to gather the ionic liquid collected by the collection sheet.

27. The apparatus of claim 26, further comprising an ionic liquid returning unit for returning the ionic liquid collected in the collection tub to the storage tank to reuse the ionic liquid.

28. The apparatus of claim 22, further comprising a recovery tub that selectively communicates with the storage tank and is removably attached to the storage tank to separately recover the recrystallized organic material.

29. The apparatus of claim 22, further comprising a bubble fining unit for reducing a volume of bubbles produced by incorporating a gas mixture into the ionic liquid of the storage tank.

30. The apparatus of claim 22, further comprising an inert gas returning unit for returning the inert gas discharged by the discharge pump to the inert gas supply source to reuse the inert gas.
